# EUROPÄISCHE PATENTSCHRIFT

(11) **EP 0 591 805 B1**
(45) Veröffentlichungstag und Bekanntmachung des Hinweises auf die Patenterteilung: **04.12.1996**
(21) Anmeldenummer: 93115555.0
(22) Anmeldetag: 27.09.1993
(51) Int. Cl.: C07D 309/06

(54) **Verfahren zur Herstellung von 4-Hydroxymethyltetrahydropyran**
Process for preparing 4-hydroxymethyl-tetrahydropyran
Procédé de préparation de tétrahydropyranne-4-hytroxyméthyl

(30) Priorität: 05.10.1992 DE 4233430
(43) Veröffentlichungstag der Anmeldung: 13.04.1994
(73) Patentinhaber: BASF Aktiengesellschaft, 67063 Ludwigshafen (DE)
(72) Erfinder: Weyer, Hans-Juergen, Dr., D-6800 Mannheim 1 (DE); Fischer, Rolf, Dr., D-6900 Heidelberg (DE); Schnurr, Werner, Dr., D-6719 Herxheim (DE); Goetz, Norbert, Dr., D-6520 Worms 1 (DE); Kuekenhoehner, Thomas, Dr., D-6737 Boehl-Iggelheim (DE)

(56) Entgegenhaltungen:
- EP-A- 0 246 581
- EP-A- 0 284 969
- EP-A- 0 546 396
- EP-A- 0 549 898
- JOURNAL OF THE AMERICAN CHEMICAL SOCIETY. Bd. 98, Nr. 20 , 29. September 1976 , GASTON, PA US Seiten 6350 - 6353 W.H. RASTETTER 'sym-Oxepin Oxide'
- JOURNAL OF ORGANIC CHEMISTRY. Bd. 32 , Januar 1967 , EASTON US Seiten 200 - 204 W.J. GENSLER ET AL. 'Rearrangement of Delta2-Dihydropyran over Hot Alumina'
- JOURNAL OF THE CHEMICAL SOCIETY, CHEMICAL COMMUNICATIONS. Nr. 18 , 15. September 1976 , LETCHWORTH GB Seiten 734 - 736 J.E. BALDWIN 'Rules for Ring Closure'

## Beschreibung

Die vorliegende Erfindung betrifft ein neues Verfahren zur Herstellung von 4-Hydroxymethyltetrahydropyran durch sauer katalysierte Isomerisierung von 3-(2-Hydroxyethyl)tetrahydrofuranen.

Aus J. Am. Chem. Soc. 98 (1976), 6350-6353 ist die Herstellung von 4-Hydroxymethyltetrahydropyran durch Reduktion von 4H-Pyran-4-carboxaldehyd bekannt.

Aus J. Org. Chem. 32 (1967), 200 bis 204 ist die säurekatalysierte Umlagerung von 2-Hydroxymethyltetrahydrofuran an Al₂O₃ zum Dihydropyran, einem Enolether, bekannt.

Aus J. Chem. Soc., Chem. Commun. (1976), 734 bis 736 ist ein allgemeiner Hinweis bekannt, sechsgliedrige Ringe durch kationische Ringschlußreaktionen herzustellen.

Es ist bekannt, daß 4-Hydroxymethyltetrahydropyran in drei Reaktionsschritten durch Umsetzung von Acetessigester mit Ethylenoxid zu 3-(2-Hydroxyethyl)-gamma-butyrolacton (EP-A-246 581), Umlagerung zu Tetrahydropyran-4-carbonsäureester (EP-A-284 969) und anschließende katalytische Hydrierung zu 4-Hydroxymethyltetrahydropyran (DE-A-41 41 222) hergestellt werden kann.

Dieser Weg zu 4-Hydroxymethyltetrahydropyran ließ durch die hohe Zahl von Reaktionsschritten zu wünschen übrig.

Der vorliegenden Erfindung lag daher die Aufgabe zugrunde, ein einfacheres Verfahren zur Herstellung von 4-Hydroxymethyltetrahydropyran zu entwickeln.

Demgemäß wurde ein neues und verbessertes Verfahren zur Herstellung von 4-Hydroxymethyltetrahydropyran gefunden, welches dadurch gekennzeichnet ist, daß man Tetrahydrofurane der allgemeinen Formel I 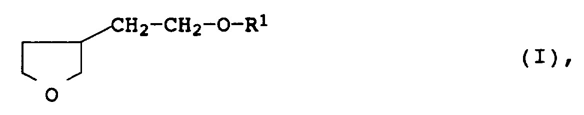 in der
- R¹: Wasserstoff, C₁- bis C₆-Alkyl oder -CO-R² und
- R²: Wasserstoff oder C₁- bis C₆-Alkyl bedeutet,
bei Temperaturen von 0 bis 400°C und Drücken von bei 0,001 bis 400 bar in Gegenwart von sauren Katalysatoren umsetzt.

Das erfindungsgemäße Verfahren läßt sich wie folgt durchführen:

Die Umsetzung von Tetrahydrofuranen I zu 4-Hydroxymethyltetrahydropyran läßt sich im allgemeinen bei Isomerisierungstemperaturen von 0 bis 400°C, bevorzugt 50 bis 200°C und besonders bevorzugt 100 bis 150°C und Drücken von 0,001 bar bis 400 bar, bevorzugt 0,01 bis 3 bar und besonders bevorzugt von 0,05 bis 0,5 bar in der Gas- oder Flüssigphase diskontinuierlich oder bevorzugt kontinuierlich in geeigneten Reaktionsgefäßen gegebenenfalls in einem inerten Lösungsmittel durchführen.

Die Substituenten R¹ und R² in der Verbindung I haben unabhängig voneinander folgende Bedeutung
R¹, R²
   - Wasserstoff
   - C₁- bis C₆-Alkyl, bevorzugt C₁- bis C₄-Alkyl wie Methyl, Ethyl, n-Propyl, iso-Propyl, n-Butoxy, iso-Butyl, sec.-Butyl und tert.-Butyl, besonders bevorzugt Methyl und Ethyl,
R¹
   - Acyl -CO-R².

Geeignete Tetrahydrofurane I sind z.B. 3-(2-Rydroxyethyl)tetrahydrofuran, dessen Hydroxyethylgruppe z.B. zum Methyl-, Ethyl-, Propyl-, Butylether verethert oder z.B. mit Ameisensäure, Essigsäure, Propionsäure, Buttersäure, n- und i-Valeriansäure, Benzoesäure verestert sein kann. Besonders bevorzugt ist 3-(2-Hydroxyethyl)tetrahydrofuran.

Als saure Katalysatoren eignen sich Homogenkatalysatoren wie Sulfonsäuren, z.B. Fluorsulfonsäure, Chlorsulfonsäure und p-Toluolsulfonsäure, Halogenwasserstoffsäuren, z.B. Iodwasserstoffsäure, Bromwasserstoffsäure und Chlorwasserstoffstoffsäure, Heteropolysäuren, z.B. Dodecawolframphosphorsäure, Dodekamolybdatophosphorsäure und Dodekawolframatokieselsäure, Carbonsäuren, z.B. Trifluoressigsäure und Trichloressigsäure, Mineralsäuren, z.B. Schwefelsäure und Perchlorsäure oder Heterogenkatalysatoren wie saure Ionenaustauscher, Zeolithe oder saure Metalloxide in Form von Trägerkatalysatoren oder in kompakter Form. Die Art des Trägermaterials ist in der Regel nicht kritisch, es können übliche Trägermaterialien wie Siliciumdioxid, Aluminiumoxide, Titandioxide, Aktivkohle, Silikate oder Zeolithe verwendet werden. Erforderlichenfalls können zur Herstellung der Katalysatoren Bindemittel oder Formhilfsmittel mitverwendet werden. werden. Bevorzugt sind stark saure Katalysatoren.

Wird ein Heterogenkatalysator verwendet, kann dieser als Suspensions- oder Festbettkatalysator eingesetzt werden.

Als Reaktionsgefäße können Reaktoren wie Rührkessel oder Rohrreaktoren eingesetzt werden. Ein Rohrreaktor mit fest angeordnetem Katalysator kann in der Sumpffahrweise oder der Rieselfahrweise betrieben werden.

Als Lösungsmittel sind beispielsweise Wasser, Alkohole, Ketone, Carbonsäuren oder Ester geeignet. Ist ein Lösungsmittel zugegen, sollte es bevorzugt einen höheren Siedepunkt als 4-Hydroxymethyltetrahydropyran aufweisen.

Da das thermodynamische Gleichgewicht deutlich auf der Seite der Tetrahydrofurane I liegt, aber 4-Hydroxymethyltetrahydropyran einen niedrigeren Siedepunkt als z.B. 3-(2-Hydroxyethyl)tetrahydrofuran besitzt, besteht eine besonders bevorzugte Ausführungsform des erfindungsgemäßen Verfahrens darin, die Isomerisierung unter ständigem Entfernen des gebildeten 4-Hydroxymethyltetrahydropyrans beispielsweise in einer Reaktionskolonne durchzuführen und so das Gleichgewicht bis zum Erhalt der gewünschten Menge an 4-Hydroxymethyltetrahydropyran zu verschieben.

3-(2-Hydroxyethyl)tetrahydrofuran kann direkt und in guten Ausbeuten durch Hydrierung von Zitronensäure (EP-A-591 795) bzw. Zitronensäurederivaten wie Zitronensäureestern hergestellt werden.

4-Hydroxymethyltetrahydropyran ist ein Zwischenprodukt für die Herstellung von Wirkstoffen, beispielsweise für den Pflanzenschutz (DE-A-31 21 355).

### Beispiele

### Beispiele 1 bis 15

Durch katalytische Hydrierung von Zitronensäuretriethylester gewonnenes 3-(2-Hydroxyethyl)tetrahydrofuran wurde durch 3-stündiges Erwärmen in Gegenwart von sauren Katalysatoren in 4-Hydroxymethyltetrahydropyran umgelagert. Für die Versuche wurde jeweils 1 g 3-(2-Hydroxyethyl)tetrahydrofuran eingesetzt.

Tabelle 1 gibt die bei verschiedenen Reaktionsbedingungen jeweils gefundenen Gehalte an 4-Hydroxymethyltetrahydropyran (4HTP) im Reaktionsaustrag wieder.

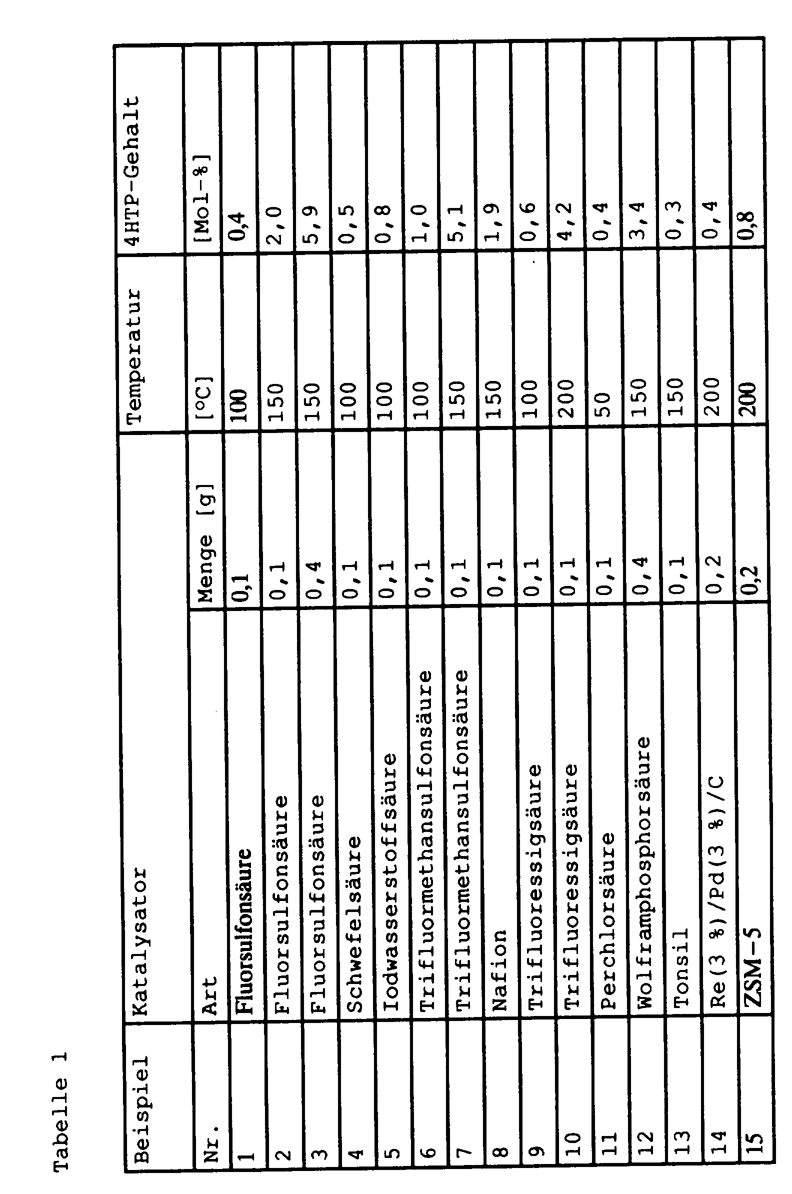

## Patentansprüche

1. Verfahren zur Herstellung von 4-Hydroxymethyltetrahydropyran, dadurch gekennzeichnet, daß man Tetrahydrofurane der allgemeinen Formel I 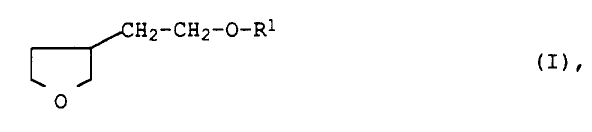 in der
R¹ Wasserstoff, C₁- bis C₆-Alkyl oder -CO-R² und
R² Wasserstoff oder C₁- bis C₆-Alkyl bedeutet,
bei Temperaturen von 0 bis 400°C und Drücken von bei 0,001 bis 400 bar in Gegenwart von sauren Katalysatoren umsetzt.

2. Verfahren zur Herstellung von 4-Hydroxymethyltetrahydropyran nach Anspruch 1, dadurch gekennzeichnet, daß man bei Temperaturen von 50 bis 200°C und Drücken von 0,01 bis 3 bar arbeitet.

3. Verfahren zur Herstellung von 4-Hydroxymethyltetrahydropyran nach Anspruch 1, dadurch gekennzeichnet, daß man bei Temperaturen von 100 bis 150°C und Drücken von 0,05 bis 0,5 bar arbeitet.

4. Verfahren zur Herstellung von 4-Hydroxymethyltetrahydropyran nach Anspruch 1, dadurch gekennzeichnet, daß man die Umsetzung kontinuierlich durchführt.

## Claims

1. A process for preparing 4-hydroxymethyltetrahydropyran, which comprises reacting tetrahydrofurans of the general formula I 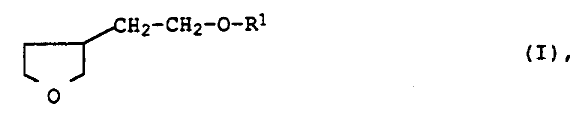 where
R¹ is hydrogen, C₁- to C₆-alkyl or -CO-R², and
R² is hydrogen or C₁- to C₆-alkyl,
at from 0 to 400°C and from 0.001 to 400 bar in the presence of acidic catalysts.

2. A process as claimed in claim 1, wherein the reaction is carried out at from 50 to 200°C and from 0.01 to 3 bar.

3. A process as claimed in claim 1, wherein the reaction is carried out at from 100 to 150°C and from 0.05 to 0.5 bar.

4. A process as claimed in claim 1, wherein the reaction is carried out continuously.

## Revendications

1. Procédé de préparation du 4-hydroxyméthyltétrahydropyranne, caractérisé en ce que l'on fait réagir des tétrahydrofurannes de la formule générale I 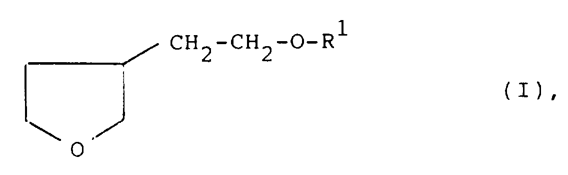 dans laquelle
R¹ représente un atome d'hydrogène, un radical alkyle en C₁ à C₆ ou -CO-R² et
R² représente un atome d'hydrogène ou un radical alkyle en C₁ à C₆,
à des températures de 0 à 400°C et sous des pressions de 0,001 à 400 bars, en présence de catalyseurs acides.

2. Procédé de préparation du 4-hydroxyméthyltétrahydropyranne selon la revendication 1, caractérisé en ce que l'on travaille à des températures de 50 à 200°C et sous des pressions de 0,01 à 3 bars.

3. Procédé de préparation du 4-hydroxyméthyltétrahydropyranne selon la revendication 1, caractérisé en ce que l'on travaille à des températures de 100 à 150°C et sous des pressions de 0,05 à 0,5 bars.

4. Procédé de préparation du 4-hydroxyméthyltétrahydropyranne selon la revendication 1, caractérisé en ce que l'on entreprend la réaction en continu.
